# EUROPEAN PATENT SPECIFICATION

(11) **EP 0 723 464 B1**
(45) Date of publication and mention of the grant of the patent: **30.06.2004**
(21) Application number: 94930390.3
(22) Date of filing: 10.10.1994
(51) Int. Cl.: A61M 5/158

(54) **SYRINGE HANDLE**
SPRITZENGRIFF
MANCHE DE SERINGUE

(30) Priority: 11.10.1993 SE 9303325
(43) Date of publication of application: 31.07.1996
(73) Proprietor: Wiklund, Ernst Sigurd Gustaf Folke, S-120 48 Enskede Gard (SE)
(72) Inventor: Wiklund, Ernst Sigurd Gustaf Folke, S-120 48 Enskede Gard (SE)
(86) International application number: PCT/SE1994/000951
(87) International publication number: WO 1995/010313

(56) References cited:
- DE-A- 4 244 653

## Description

In the medicine there is frequently need for establishing connection between blood vessels (veins and arteries) and injectors or containers of different kinds. The connection must be erected with total sterility. Leakage must be avoided as far as possible. Examples of treatments requiring blood vessel contact erection are blood transfusion inclusive blood giving, blood sampling with the assistance of test containers under negative pressure, and intravenous supply of medicine, nourishment or just liquid for blood volume expansion, commonly called drip. In all these applications it is essential that the puncture of the vessel wall can be done without damage to the surrounding tissues. Failures are even under the best circumstances unpleasant, but may cause serious damages as well.

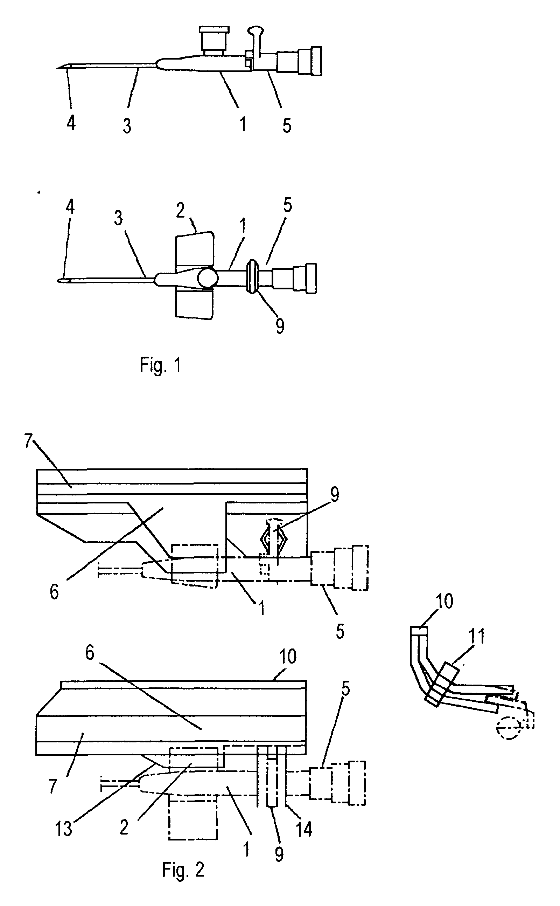

A common syringe for insertion in blood vessels of different kinds is shown in figure 1. The syringe consists of a tube body (1) with a channel that continues in a thin plastic tube (3). The rear opening of the tube body is designed with a conical widening for attachment to a syringe or a catheter. The opening is, at the beginning, filled by the hollow needle (4), which continues with the needle body (5), the rear part of which has a transparent room, which gets filled with blood, when satisfactory blood vessel contact has been attained. Through the channel runs the hollow puncture needle (4) with an, at oblique angle, sharpened tip, which extends a few millimetres in front of the front end of the thin plastic tube.

When establishing a blood vessel connection one chooses a suitable superficial blood vessel (vein or artery depending upon type of treatment) and tries to direct the tip of the puncture needle to penetrate the skin just over the chosen blood vessel so that the tip hits the vessel centrally and, after penetration of the vessel wall, together with the plastic tube can be directed axially obliquely into the blood path. When the above mentioned transparent room behind the needle starts filling with blood the needle body, with the belonging puncture needle, is kept still, while the tube body is advanced forward, so that the needle tip no longer is in front of the end of the plastic tube. This is done to prevent the needle tip from hurting the vessel wall, when the plastic tube afterwards to the main part of its length is brought forward into the blood path with the aid of the tube body. When the plastic tube has reached the desired position in the blood path, the tube body is kept still. The puncture needle, which so far has prevented leakage by its close fit to the inner wall of the plastic tube, is withdrawn with the needle body and the desired connection to syringe, container or catheter is erected.

For reasons of sterility the plastic tube must not be touched before or during the insertion into the blood lumen. The distance from the needle tip to the grip around the needle body is long. The grip surfaces are not well defined, especially as the hands most frequently have to be provided with gloves. The insertion demands a row of complicated changes of direction. There is no visible indication of the position of the needle tip after the skin penetration. To this is added that the blood vessels are frequently relatively badly fixed under the skin and are having big tendencies to "roll away".

In spite of these, from the ergonomic point of view, very unfavourable circumstances experienced nurses with daily training develop great skill and fail very seldom. The circumstances are different for doctors, nurses, ambulance crews and learners, which more temporarily are put to the task. Failures will cause the patient discomfort and are embarrassing to the operator. Besides loss of valuable material will arise.

Amendment proposals concerning the ergonomics at the establishment of vein and artery connections and similar operations can be found in the German "Offenlegungsschriften" 2306068 and 4244563. The solution of the problem according to the former is applicable to injection needles without surrounding plastic tubes only. It is true that the latter one mentions the possibility of combination with a plastic tube, but the proposed design involves large risks of damages as no firm connection between the needle body and the tube body exists. As will be explained below unintended displacements between those parts involve serious moments of risk.

DE-A-4244653 proposes the use of a slotted handle. The slot is used for the location of the wing of a one-winged tube body. Obviously two options are possible.
1. Making the slot narrow compared with the wing dimensions. In that way a sort of connection may be obtained but separation of the two bodies after the penetration phase will be very difficult.
2. Making the slot wide compared with the wing dimensions. This gives no connection at all.

The applicant behind the DE-A-4244653 seems to have been aware of the problem but his proposal, making the handle elastic and using finger pressure to compensate for loose fitting, will only make separating even more difficult as the handle has to be let loose during the separation procedure.

The invention concerns a handle (6) with a grip arrangement (7) for a syringe consisting of a tube body (1) with a thin plastic tube (3) that surrounds a hollow puncture needle (4) fastened in a needle body (5), intended for blood vessel puncture attaining blood path connection. The handle (6) is adapted to be connected both to the tube body (1) and to the needle body (5) before and during the puncture phase, but is after the puncture phase easily disengageable from the tube body (1), which is moved forward, when blood path contact has been obtained. The grip arrangement (7) permits thumb/forefinger placement at the side of and close to the tip of the puncture needle of said syringe. The handle can be disengaged from the tube body (1) with preserved thumb/forefinger grip.

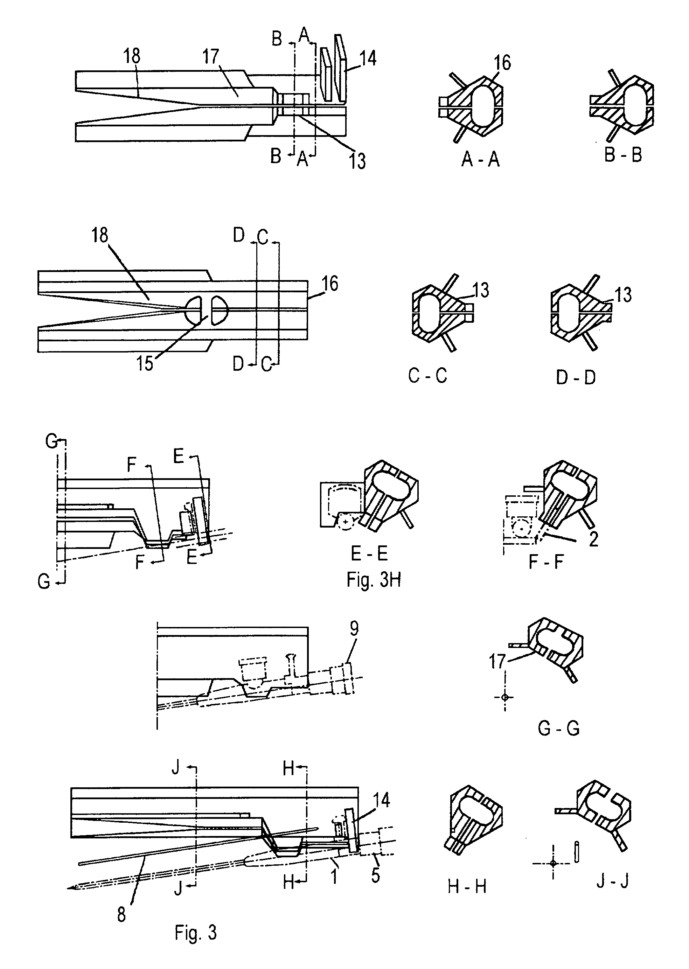

in preferred ways of carrying out the invention the grip part (7) of the handle is designed so that other parts of the hand can help the governing of the syringe. Examples of such embodiments of the invention are shown in figures 2 and 3.

When the tube body has been disengaged, it may by the other hand of the operator be advanced into the lumen to a suitable position without changing the position of the needle body and with it the puncture needle position. The handle may be provided with an indication stick (8) that indicates the position of the tip of the puncture needle and may be of good assistance to an inexperienced operator.

When the tube body (3) has been advanced to the desired position, the puncture needle (4) is removed. To prevent blood flow through the tube body's rear end, which has been sealed by the needle body, the operator begins the removal by compressing the skin area above the front end of the plastic tube with his/hers other hand, until he or she has withdrawn the puncture needle let go the handle with the puncture needle and placed the for this purpose intended stopper in the rear end of the tube body. Now the operator has both hands free to fix and flow test the tube body and separate the needle body (5) from the handle, which may be taken care of for cleaning, autoclaving and re-packing.

The handle grip (7) must, besides being ergonomically friendly, provide a comfortable but firm and stabile fixation to the needle body (5), suitably to the shield (9), and also to the tube body (1) suitably to one of its wings (2). In this way a sure protection against unintended displacement of the needle tip relatively to the tube end is obtained. The needle tip is very sharp. At unsuitable displacements of their mutual position the sharpened edges may cut loose plastic fragments from the tube tip. The fragments may get into the blood path. This may have incalculable consequences.

When the handle grip has been mounted, the operator may begin a skin and vessel puncture after the usual hygienic preparations. The thumb/ forefinger grip in the proximity of the needle tip is ergonomically favourable and permits the fine motor movements of the hand coming to their rights. The bigger muscle groups in the under arm, upper arm and trunk need normally not take part. The person who so wishes may get guidance at and after the vessel wall penetration by the indication stick (8), with which the handle may be provided.

A handle with the qualities mentioned above may be designed in several different ways. A couple of those are shown in figures 2 and 3.
Fig. 1 is showing two parallel projections in two against each other perpendicular planes of a so-called VENFLON ^{(R)}-syringe, which is suitable for use with the handle according to the invention. VENFLON is a registered trademark.
Fig. 2 and 3 are showing examples of different preferred ways of carrying out the invention.

Figure 2 shows a handle (6) consisting of two straight beams both with an almost uniformly curved section and joined by a border (10) of a suitable polymer material with springy hinge function and with a snap lock (11) at the back end. The beams are designed to enclose one of the wings of the tube body (2) and fix it with the aid of the snap lock (11). One of the beams, preferably the one below, is provided with a projecting squeeze arrangement (14) suitable to be placed over the shield (9) of the needle body (5) from the side or from above, at which the rear half of the squeeze arrangement first comes into contact with the back surface of the shield to prevent tendencies for backward movement of the needle body, and the shield in the squeezed position becomes fixed not just in height and side position but also in a defined direction essentially parallel to the squeeze arrangement (14). With preserved thumb/forefinger grip the snap lock (11) shall allow opening with the little or ring finger, at which the tube body's wing (2) is let free and the tube body may be advanced while the needle body still may be controlled and manipulated by the preserved thumb/forefinger grip.

Figure 3 is showing a handle constructed as a hollow hexagonal rod with a function that reminds of a clothes nip. The hexagonal rod is, with the exception of the piece (15) of the back wall (16), which serves as a springy hinge, cut longitudinally through the front wall (17) and the mentioned back wall (16). The front part of the rod is cut obliquely to form shanks (18) of the clothes nip. The shank angles may preferably be larger at the front side (17) in order to give a somewhat larger opening of the rear front wall cut of the rod. The rear part of the front wall (17) of the rod has a pair of projecting jaws (13) preferably provided with one or more teeth intended to fix one of the wings of the tube body firmly. The rear part of the rod has on one, relatively the longitudinal cut, upper wall a squeeze arrangement (14) suitable for the shield (9) of the needle body. The rear half of the squeeze arrangement is made to get into contact with the rear wall of the shield (9) to prevent tendencies of the needle body to move backwards relatively to the tube body. The squeeze arrangement (14) is designed to give a firm fixation of the shield for the desired needle direction as well as height and side.

The design provides that one, with an unchanged thumb/forefinger grip, first can securely direct the skin and blood vessel puncture, secondly can open the clothes nip to let the tube body wing free and with ease advance the tube body, while the needle body is still fixed in the handle in unchanged position, until the needle body can finally be withdrawn and the rear end of the tube body made tight by the stopper that until now has been sitting at the rear end of the needle body.

The figure is showing the possibility of attaching the earlier mentioned indication stick (8) to indicate the position of the needle tip and also profiles with the object first to prevent the gloves of the operator from contaminating the plastic tube or the needle tip secondly to reinforce the shanks of the clothes nip. In the example a hexagonal rod has been chosen and this is one, from the grip point of view, preferred design. However, other polygonal and circular crosscuts are just as useful even if less grip friendly.

A handle according to the invention may be designed as a single-use article. Then nothing prevents designing it as permanently bound to the needle body and temporarily, and easily releasable, to the tube body. As the invention primarily is intended as expedient to operators that due to deficient training otherwise may have difficulties to perform blood vessel punctures in the correct way designing as a separate attachment and multi-use article is preferred.

Attaching a multi-use handle according to figure 3 to a syringe is done as follows: The rear half of the squeeze arrangement (14) is brought into contact with the back side of the shield (9) and thus preventing backward movements of the puncture needle (4) relatively to the plastic tube (3). Thereafter the jaws (13) that are to enclose one of the wings (2) of the tube body are opened by pressing together the shanks (18). The wing is brought into the gap between the jaws (18) and fixed there by letting go of the pressure on the shanks (18). During the entire procedure is observed that the rear half of the squeeze arrangement rests against the backside of the shield. After the fixation of the tube body's wing the syringe is turned around an axis, which is roughly parallel to the axis of the syringe and runs through the joint of the wing (2) to the tube body, until one reaches the desired grip position at the same time as the squeeze arrangement (14) is brought to its final position at the needle body (5) and fixed there. Mounting of a handle according to figure 2 and variants of the exemplified handles is done correspondingly as far as applicable.

## Claims

1. Handle (6) with a grip arrangement (7) for a syringe, said syringe consisting of a tube body (1) with a thin plastic tube (3) that surrounds a hollow puncture needle (4) fastened in a needle body (5), and intended for blood vessel puncture and for attaining blood path connection; the handle (6) being adapted to be connected both to the tube body (1) and to the needle body (5) before and during the puncture phase, but after the puncture phase being easily disengageable from the tube body (1), which is moved forward, when blood path contact has been obtained, ***characterized in that*** said grip arrangement (7) permits thumb/forefinger placement at the side of and close to the tip of the puncture needle of said syringe and **in that** said handle can be disengaged from the tube body (1) with preserved thumb/forefinger grip.

2. The handle of claim 1 ***characterised in that*** the handle is of multi-use type and designed as a separate autoclave-able unit for rapid attachment to the tube body (1) and the needle body (5).

3. The handle of claim 1 ***characterised in that*** the handle is of single-use type and may be integrated with the needle body (5).

4. The handle of claim 1 ***characterised in that*** the handle is provided with an indication stick to indicate the tip position of the needle.

5. Method of mounting a multi-use handle according to claim 1 and 2 whereas the needle body has a shield (9), the handle has a squeeze arrangement (14) and jaws (13), and the tube body has wings (2) ***characterised in that*** rear half of the squeeze arrangement (14) is brought and kept into contact with the back side of the shield (9) until one of the wings (2) of the tube body has been fixed between the jaws (13), thereafter the syringe is turned around an axis, which is parallel to the syringe axis and runs through the joint of the wing (2) to the tube body, until one reaches the desired grip position of the handle at the same time as the squeeze arrangement (14) is brought to its final position and in this way is fixed to the shield (9) of the needle body.

## Patentansprüche

1. Handhabe (6) mit einem Griffarrangement (7) für eine Kanüle, besagte Kanüle bestehend aus einem Rohrkörper (1), der mit einem dünnen flexiblen Rohr (3) versehen ist, das Rohr eine hohle Nadel (4) umgebend, die an einem Nadelkörper (5) angeschlossen ist, beabsichtigt für Blutgefässpunktierung und für die Aufrichtung von Blutgefässverbindung; die Handhabe (6) ist so ausgeformt, daß sie sowohl an dem Rohrkörper (1) als an dem Nadelkörper (5) vor und während der Punktierungsphase angeschlossen ist, aber nach der Punktierungsphase leicht von dem Rohrkörper (1) getrennt werden kann, der nach vorn versetzt wird, wenn Blutgefässkontakt erhalten ist, ***dadurch gekennzeichnet,* daß** besagtes Griffarrangement (7) Daumen/Zeigefingerplazierung neben der Seite von und nahe zur Spitze der Punktierungsnadel der besagten Kanüle erlaubt und dadurch, daß die besagte Handhabe mit beibehaltenem Daumen/Zeigefingergriff von dem Rohrkörper (1) getrennt werden kann.

2. Die Handhabe des Anspruchs 1 ***dadurch gekennzeichnet,* daß** die Handhabe von Wiederverwendungstype ist und ausgeformt als eine separate sterilisierbare Einheit für Schnellanschluss zum Rohrkörper (1) und dem Nadelkörper (5) ist.

3. Die Handhabe des Anspruchs 1 ***dadurch gekennzeichnet,* daß** die Handhabe von Einmalverwendungstype ist und mit dem Nadelkörper (5) integriert sein kann.

4. Die Handhabe des Anspruchs 1 ***dadurch gekennzeichnet,* daß** die Handhabe mit einem Indizierungshorn zum Indizieren der Spitzposition der Nadel versehen ist.

5. Verfahren für das Montieren einer Wiederverwendungshandhabe laut Anspruchs 1 und 2 wobei der Nadelkörper ein Schild (9) hat, die Handhabe ein Quetschungsarrangement (14) und Klauen (13) hat, und der Rohrkörper Flügel (2) hat, ***dadurch gekennzeichnet,* daß** eine hintere Hälfte des Quetschungsarrangements (14) gebracht und in Kontakt mit der Hinterseite des Schilds (9) gehalten wird bis einer der Flügel (2) des Rohrkörpers zwischen den Klauen(13) festgemacht worden ist, danach die Kanüle um eine Achsel herumgredreht wird, die parallel zu der Achsel der Kanüle ist und läuft durch das Gelenk des Rohrkörperflügels (2), bis die gewünschte Griffposition der Handhabe erreicht wird, gleichzeitig wie das Quetschungsarrangement (14) zu seiner Endposition gebracht und in dieser Weise an dem Schild (9) des Nadelkörpers festgemacht wird.

## Revendications

1. Poignée (6) avec un arrangement de prise (7) d'une canule, la canule dite composant d'un corps de tube (1) avec un tuyau mince et souple (3) entourant une aiguille de ponction creuse (4) connectée à un corps d'aiguille (5), fait pour ponction des vaisseaux sanguin et pour obtenir connexion du cours de sang; la poignée (6) est adaptée pour être connectée au corps de tube (1) et au corps d'aiguille (5) avant et pendant la phase de la ponction, mais après la phase de ponction est facile de libérer du corps de tube (1), lequel est avancé, quand une connexion du cours de sang est établie, ***caracterisée en* ce que** l'arrangement de prise (7) dite permit pouce/index placement au côté de et tout près au bout de l'aiguille de ponction de la canule dit et **en ce que** la poignée dite est possible de libérer du corps de tube (1) avec la prise de pouce/index conservé.

2. La poignée de la revendication 1 ***caracterisée en ce* que** la poignée est du type usage répété, et façonnée comme une unité séparée et autoclavable pour le montage rapide au corps de tube (1) et corps d'aiguille (5)

3. La poignée de la revendication 1 ***caracterisée en ce* que** la poignée est du type article à jeter, et possible d'intégrer avec le corps d'aiguille (5)

4. La poignée de la revendication 1 ***caracterisée en ce* que** la poignée est équipée avec une baguette d'indication pour indiquer la position du bout de l'aiguille.

5. Methode du montage d'une poignée du type usage répété selon des revendications 1 et 2 où le corps d'aiguille a un bouclier (9), la poignée a un arrangement de presser (14) et mâchoires (13), et le corps de tube a des ailes (2) ***caracterisée en ce* que** la moitié arrière de l'arrangement de presser (14) est mené et teni en contact avec le côté derrière du bouclier (9) jusqu'à une des ailes (2) du corps de tube est fixée entre les mâchoires (13), ensuite la canule est tornée en rond un axe, qui est parallèle à l'axe de la canule et va par la jointure de l'àile (2) du corps de tube, jusqu'à on arrive à la position de prise desirée en meme temps que l'arrangement de presser (14) est mené à sa position definitive, et fixé au bouclier (9) du corps d'aiguille à cette manière.
